(19)

Europäisches
Patentamt
European
Patent Office
Office européen
des brevets

(11) **EP 2 836 175 B1**

(12) **EUROPEAN PATENT SPECIFICATION**

(45) Date of publication and mention
of the grant of the patent:
**27.03.2019 Bulletin 2019/13**

(21) Application number: **13702016.0**

(22) Date of filing: **29.01.2013**

(51) Int Cl.:
***A61F 9/008*** *(2006.01)*

(86) International application number:
**PCT/EP2013/051616**

(87) International publication number:
**WO 2013/152875 (17.10.2013 Gazette 2013/42)**

(54) **LASER DEVICE AND PROCESS FOR CONFIGURING SUCH LASER DEVICE**

LASER VORRICHTUNG UND VERFAHREN ZUM KONFIGURIEREN EINER DERARTIGEN
LASERVORRICHTUNG

DISPOSITIF LASER ET PROCÉDÉ DE CONFIGURATION D'UN TEL DISPOSITIF LASER

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.04.2012 DE 102012007272**

(43) Date of publication of application:
**18.02.2015 Bulletin 2015/08**

(73) Proprietor: **WaveLight GmbH
91058 Erlangen (DE)**

(72) Inventors:
• **VOGLER, Klaus
99444 Blankenhain (DE)**
• **DONITZKY, Christof
90542 Eckental (DE)**
• **WUELLNER, Christian
91094 Bräuningshof (DE)**

(74) Representative: **Katérle, Axel
Wuesthoff & Wuesthoff
Patentanwälte PartG mbB
Schweigerstraße 2
81541 München (DE)**

(56) References cited:
**EP-A1- 2 345 394          US-A- 5 520 679
US-A1- 2004 243 111**

• **COLOMBELLI JULIEN ET AL: "Ultraviolet
diffraction limited nanosurgery of live biological
tissues", REVIEW OF SCIENTIFIC
INSTRUMENTS, AIP, MELVILLE, NY, US, vol. 75,
no. 2, 1 February 2004 (2004-02-01), pages
472-478, XP012071303, ISSN: 0034-6748, DOI:
10.1063/1.1641163**

Printed by Jouve, 75001 PARIS (FR)

**Description**

The present disclosure is concerned in one aspect with a laser device for use in human eye surgery. In another aspect, the present disclosure is concerned with configuring a laser device so that with the latter a two-dimensionally extensive separation of tissue in human corneal or lenticular tissue is achievable by stringing together a plurality of local sites of damage.

The separation of tissue (surface of incision) may be required in a variety of laser-assisted treatments of the human cornea or lens, for instance for the generation, by means of laser technology, of a lamella of tissue (ordinarily designated in specialist terminology as a flap) that is capable of being folded away on the anterior side of the cornea within the scope of a LASIK treatment (LASIK: laser in-situ keratomileusis) or for cutting away, by means of laser technology, certain areas of the corneal tissue within the scope of a keratoplasty (ordinarily designated in specialist terminology as a cap) or for capsulorhexis within the scope of a cataract treatment of the human lens. It will be understood that these are only examples of forms of treatment in which a laser device configured in accordance with the present disclosure may come into operation. Other laser-assisted forms of treatment of the human cornea or lens are by no means excluded.

Pulsed laser radiation with a pulse duration within the three-digit femtosecond range has been used for the generation of tissue-separating incisions in the human cornea. However, suitable laser systems which are able to make available radiation pulses with such short pulse durations are comparatively complex, susceptible to interference and expensive. It has, however, been a widespread view hitherto amongst experts that for intraocular incisions in which utmost precision and reproducibility of the generation of an incision is what matters to a quite particular degree, only laser pulses within the femtosecond range are suitable. With respect to the state of the art regarding the machining of the human cornea with fs laser pulses, reference may be made in exemplary manner to EP 1 787 607 A1, US2004/0243111 and US5520679. A laser machining of a transparent material with pulse durations within the nanosecond range is known from EP2345394, machining below the threshold of optical breakdown is disclosed, for example, in WO 2008/151616 A1.

It is an object of the invention to provide a laser device usable for laser-assisted treatments of the human eye, wherein the laser device can be realised with comparatively little complexity and inexpensively.

With a view to achieving this object, the present disclosure provides in one aspect a process for configuring a laser device that is adapted for emitting focused pulsed laser radiation and that is intended for use in laser-assisted treatments of the human eye. The process comprises the following steps: selecting a pulse length for the laser radiation within a range from above 10 ps to below 300 ps; selecting a wavelength for the laser radiation within a range from 300 nm to 400 nm or within a range from 800 nm to 1100 nm; performing experiments on PMMA test plates to ascertain such a set of parameter values of the laser device such that when the laser device is operated with these parameter values a two-dimensionally extensive tissue separation of human corneal or lenticular tissue is achievable by means of the laser radiation by stringing together a plurality of local sites of damage, each local site of damage including a laser induced optical breakdown of the tissue, the set of parameter values including, in addition to the selected pulse length and the selected wavelength, values for at least one of the following parameters: a pulse energy, a pulse repetition rate, a fluence per radiation pulse, a number of pulses per site of damage, a scanning speed of a scanning apparatus of the laser device, a focus diameter; and configuring the laser device in such a manner that operation of the laser device with the ascertained set of parameter values is enabled, wherein the set of parameter values is ascertained for a plurality of radiation pulses per site of damage and the fluence per radiation pulse is selected below a fluence threshold for single-pulse damage.

The ascertaining of the set of parameter values may include, for example, the implementation of experimental tests on test plates made of PMMA (poly methyl methacrylate) in order, on the basis of a selected pulse length and a selected wavelength, to find suitable values for one or more of the further named parameters, so that in the test material permanent damage occurs which - given manifold juxtaposition of such sites of damage - can be utilised for the purpose of generating a two-dimensionally or three-dimensionally shaped surface of incision in the ocular tissue. One or more of the parameter values thus ascertained may be adopted, unchanged or virtually unchanged, for the configuration of the laser device, such as when the test material, is well comparable to the human eye with regard to its interaction with laser radiation. Further, one or more of the experimentally ascertained parameter values may be adjusted in the course of the configuration of the laser device by suitable correction factors. Such correction factors may be ascertained beforehand or may be generally known in the art and may e.g. be taken into consideration in order, if desired, to take into account certain peculiarities of the human eye that have been ascertained from, for example, postoperative analyses.

The configuration of the laser device may, for example, be such that the selected pulse length or/and the selected wavelength is/are set permanently in the laser device and can no longer be changed by the user. The remaining parameters of the parameter set may be capable of being set, at least in part, by the user, in which connection the available ranges of adjustment include the parameter values ascertained within the scope of the ascertainment step. As far as the breadth of these ranges of adjustment is concerned, one possibility consists in defining at least a fraction of the ranges of adjustment to be so narrow that the damage procedure in the human corneal or lenticular tissue required for the separation of tissue is achievable within the entire range of adjustment. In this manner the user can be relieved of the uncertainty as to whether a change of setting performed by him/her will possibly no longer trigger the desired damage process in the ocular tissue. Of course, it should not be ruled out that at least a fraction of the ranges of adjustment is, in contrast, so large that the desired damage process in the ocular tissue does not arise within the entire range of adjustment, given unchanged values of the other parameters.

The set of parameter values is preferably ascertained in such a manner that at each site of damage a laser-induced optical breakdown of the tissue is achievable. With this configuration each site of damage includes a photodisruptive local destruction of the ocular tissue, which under certain circumstances may be accompanied by a thermal effect or some other effect of the picosecond laser pulses (e.g. a change in the refractive index or a defect associated with electronic imperfections).

For each site of damage, working is proceed with a plurality of laser pulses may be beamed onto substantially the same location in the tissue in order to generate a site of damage there. In the case of multiple-pulse damage, for the ascertainment of the set of parameter values the fluence per radiation pulse is selected below a fluence threshold for single-pulse damage, for example within the range from 0.1 $J/cm^2$ to 1 $J/cm^2$. It should be added that for multiple-pulse damage at least one of the energy and intensity may be the same for all pulses of the burst of pulses irradiated onto substantially the same tissue location. Alternatively, at least one of the energy and intensity may vary among the pulses of a burst. For example, the energy or intensity distribution in the burst may be such that an earlier pulse of the burst has lower energy or intensity than a later pulse of the same burst.

A burst of pulses may be sub-divided into two or more sub-bursts, each sub-burst consisting of one or more laser radiation pulses. Subsequent sub-bursts may be temporally spaced from each other by more than the mutual temporal separation of subsequent pulses within a sub-burst, thereby creating a rest period between subsequent sub-bursts. The number of pulses per sub-burst may be the same among the sub-bursts of a burst, or may differ for at least some of the sub-bursts of a burst. Further, while the energy and/or intensity of pulses within a sub-burst may be substantially constant, the pulse energy and/or intensity may vary between the sub-bursts. For example, a burst causing a single site of damage may comprise a first sub-burst of relatively lower pulse energy and/or intensity, followed by a first rest period, followed by a second sub-burst of relatively higher pulse energy and/or intensity, followed by a second rest period, followed by a third sub-burst of relatively lower pulse energy and/or intensity, wherein the pulse energy and/or intensity of the third sub-burst may be lower than, equal to, or larger than the pulse energy and/or intensity of the first sub-burst.

For the ascertainment of the set of parameter values the pulse energy may be selected, for example, within the range from 1 nJ to 10 µJ, preferably within the range from 100 nJ to 1 µJ. The rate of repetition of the laser pulses emitted onto the eye may for this purpose be selected, for example, within the range from 10 kHz to 1 GHz, preferably within the range from 100 kHz to 100 MHz.

The use of ps laser pulses instead of fs pulses permits a considerably less complex and expensive laser device to be used. For example, the laser device for generating the laser radiation may be equipped with a mode-coupled or Q-switched semiconductor laser or solid-state laser or with a microchip laser or with a vertical-cavity surface-emitting laser (abbreviated to VCSEL) or with a vertical-external-cavity surface-emitting laser (abbreviated to VECSEL) or with a mode-locked integrated external-cavity surface-emitting laser (abbreviated to MIXSEL). The use of ps pulses of a mode-coupled laser permits, for example, the measures for pulse-length management (e.g. stretchers, compressors), which are otherwise present conventionally in the case of a mode-coupled fs laser, to be dispensed with.

In another aspect, the present disclosure provides a laser device for human eye surgery, comprising: a source for providing a beam of laser radiation; a scanner for scanning the beam of laser radiation; focusing optics for focusing the beam of laser radiation; a control program representing an incision figure to be generated in a human eye; and a control device coupled to the scanner for controlling the scanning of the beam of laser radiation in accordance with the control program; wherein the source includes one of the following for generating the beam of laser radiation: a mode-coupled semiconductor laser, a mode-coupled solid-state laser, a Q-switched semiconductor laser, a Q-switched solid-state laser, a microchip laser, a vertical-cavity surface-emitting laser, a vertical-external-cavity surface-emitting laser, a mode-locked integrated-external-cavity surface-emitting laser; wherein the laser radiation provided by the source has radiation parameter values permitting a two-dimensionally extensive separation of tissue of human corneal or lenticular tissue by means of the laser radiation by stringing together a plurality of local sites of damage, each local site of damage including a laser induced optical breakdown of the tissue, the laser radiation having a pulse length within a range from 10 ps to below 300 ps, and a wavelength within one of a range from 300 nm to 400 nm and a range from 800 nm to 1100 nm. nm , and wherein the radiation parameter values are set for a plurality of radiation pulses per site of damage and the fluence per radiation pulse is set below a fluence threshold for single-pulse damage.

Further elucidations with respect to the backgrounds of the invention will be given in the following.

For cutting laser applications in human ophthalmology, in particular in connection with refractive surgery and cataract surgery, fs laser systems have hitherto been employed almost exclusively. The fs laser sources that are used in these systems are often fs MOPA sources which consist of a compact fs oscillator (pulse energy ≤ 10 nJ, pulse repetition frequency > 50 MHz) and of a regenerative amplifier module. MOPA stands for master oscillator power amplifier; such amplifiers may, for example, accomplish an amplification of the pulse energy to 10 μJ to 50 μJ at a pulse frequency of over 100 kHz.

At a repetition-rate from about 40 kHz to 1 MHz (achieved by a so-called pulse picker), pulse energies from about 1 μJ to 15 μJ can be obtained by amplification of the selected oscillator pulses, said pulse energies being optimised for the designated ophthalmological applications. For industrial applications, use may even be made of pulse energies ≥ 1 mJ.

Established applications of such fs laser systems are, for example in the case of refractive surgery, the realisation of the LASIK flap incision, the implementation of various types of keratoplasties (e.g. penetrating, lamellar posterior or anterior keratoplasties) and also the generation of intrastromal annular tunnel incisions for the purpose of implanting INTACs. In the case of cataract surgery, fs laser systems are employed, for example, for the purpose of realising capsulorhexis (initial anterior capsular incision of the lens), for pre-fragmentation of the human cataract lens, for the purpose of generating lateral, scleral incisions for the insertion of the instruments in following manual phases of the cataract operation and for the purpose of generating limbal relaxation incisions with a view to avoiding an induced astigmatism.

The use of fs laser systems in such fields of application has in the meantime become routine to a certain extent. In addition, further applications of fs laser pulses are being researched, for instance for the treatment of presbyopia or of a glaucoma or for an fs refraction correction realised solely with fs laser pulses, e.g. corneal lenticle extraction.

In addition to fs sources on the basis of discrete solid-state technology with regenerative amplifier, at the present time fs fibre lasers with CPA amplifier systems (CPA: chirped pulse amplification) or HP LC oscillators (HP LC: high-power long-cavity) are also occasionally in use. Although HP LC oscillators do not require a separate amplifier stage for increasing the laser-pulse energy, their structure is also relatively complex, due, for example, to the realisation of the requisite resonator length L and/or of a cavity dumping for the purpose of coupling out laser pulses of suitable energy of, for example, above 100 nJ at a repetition-rate f ≥ 10 MHz.

These fs sources elucidated so far are generally NIR sources, that is to say, sources that radiate in the near-infrared region, for example within the range between 1000 nm and 1100 nm. By means of the third harmonic of such NIR fs laser sources, ophthalmological treatments may also be realised within the UV region. For this purpose a UV wavelength between 340 nm and 360 nm, for example, is employed. The advantage of such a UV wavelength is that distinctly lower pulse energies of the fs laser pulses within the range from a few 10 nJ to 100 nJ (at f = 200 kHz to 1000 kHz) may suffice for the same applications, that furthermore by virtue of the shorter wavelength a higher accuracy (smaller focus dimensions) can be obtained, and that, lastly, the retina of the patient is burdened less by the lower laser-pulse energies and by the absorption of the shortwave laser radiation, especially in the human lens and in the vitreous body.

However, UV fs laser systems also possess the same fundamental complexity as NIR fs laser systems, which is increased even further by virtue of the necessary two-stage frequency conversion. UV fs laser systems are to be found within the same price range as, or even within higher price ranges than, NIR fs laser systems and can be just as voluminous and susceptible to interference. The only significant advantage of the UV wavelength in comparison with the NIR wavelength is that, by virtue of the more efficient absorption of the UV radiation in the human ocular tissue in comparison with the NIR radiation, the laser-induced optical breakdown (LIOB for short) which is desired for generation of an incision in the transparent ocular tissue can be obtained already at comparatively low laser-pulse energies. This is connected with the fact that for multi-photon absorption in the case of UV laser pulses with, for example, a wavelength of 345 nm only a two-stage non-linear process is necessary, whereas in the case of NIR laser pulses with, for example, a wavelength of 1064 nm the order of the non-linearity has to be at least six-fold in order to raise the initial electrons by means of multi-photo ionisation above a band gap of about 6.5 eV (corresponds to the ionisation energy of water) into the quasi-free conduction band. In comparison with a multi-photon ionisation within the UV region, for the optical breakdown in the NIR region significantly higher intensities (pulse energies) are therefore necessary.

A further factor is presumably a slight linear residual absorption of the UV radiation (e.g. in the cornea), as a result of which the free electrons initially needed for the start of the avalanche ionisation, which otherwise have to be supplied exclusively by the multi-photon absorption, are already made available, in part, by a linear ionisation. This assists the 'ignition process' of the laser-induced breakdown in positive manner. In the visually transparent ocular tissue such as the human lens, in which the linear absorption at 300 nm to 400 nm is not negligible, this linear absorption can assist, under certain circumstances still more advantageously, the generation of the initial electrons which are necessary for the optical breakdown and for the following generation of plasma leading to the separation of tissue.

The invention makes it possible to reduce the effort required for the generation of an LIOB in human ocular tissue, by various parameters of a laser device for the stated ophthalmological applications being combined in such a way that they can be made available from simple laser sources and nevertheless achieve good results of application which are comparable to those which are possible with fs pulses. The essential central idea for this is the use of ps pulses, that is to say, laser pulses within the picosecond range, preferably within the range from above 10 ps to below 300 ps.

The development of microchip lasers and of other comparably compact short-pulse laser sources (with pulse durations from about 1 ps to 1 ns) meanwhile permits this, so that by suitable interaction of certain laser parameters the necessity for elaborately generated fs pulses, and the associated complexity, can be dispensed with.

The invention takes advantage of various physical effects that are based on different physical dependences and that act in combination in such a way that they yield determinate, comparatively low LIOB thresholds which otherwise are only obtained with ultra-short fs laser pulses.

One problem of the fs laser systems employed hitherto for ophthalmological treatments of the human eye is that the fs laser sources used therein are complex and consequently comparatively susceptible to interference and, in addition, are comparatively sensitive to the ambient conditions (e.g. outside temperature). In addition, they are of comparatively large construction, heavy and expensive. They are a quite essential cost factor of the entire treatment system and crucially determine the availability of the treatment systems in everyday clinical practice.

Hitherto in specialist circles and also in the relevant literature the position has often been advocated that only with fs laser pulses are the necessary precision and reproducibility obtained in connection with the photodisruption on which the entire generation of an incision in the case of intraocular application has hitherto been based. The investigations in this regard, however, related for the most part only to NIR wavelengths.

Experimental results of many years provide evidence that with fs pulses the lowest fluence values F=E/A (pulse energy E per surface area A, where A is proportional to the square of the focus diameter $d_F$, that is to say, $A \sim d_F^2$) are needed and at the same time the lowest collateral damage (e.g. thermal damage) to the surrounding tissue arises, since the fs pulse length is significantly shorter than the thermal-diffusion time of the tissue. The zone of interaction of the laser radiation with the tissue (expressed, for example, by an interaction diameter) is of the order of magnitude of the theoretical focus diameter $d_F$, though it is initially limited only to a range $d_N$ where the intensity (fluence) exceeds the LIOB threshold $F_{th}$. This range $d_N$ of the non-linear absorption is smaller than the extent of the focus $d_F$ and is also smaller than the extent dp of the following plasma or even the extent $d_G$ of the ultimate destruction of tissue. For wavelengths within the range of transparency of human ocular tissue, in the case of influence of fs pulses and subsequent photodisruption, as a rule the following inequality holds:

$$d_N \leq d_F < d_P < d_G \approx 10\ \mu m \text{ (depending on the wavelength)}$$

Hitherto it has often been argued that only laser pulses with pulse lengths within the range from milliseconds to nanoseconds produce thermal interactions, but that fs laser pulses act absolutely athermally, that is to say, only photodisruptively. Meanwhile it has been recognised that fs laser pulses may also leave a thermal effect behind, specifically in the course of the photodisruption alone, or even below the LIOB threshold. The region $d_T$ of the thermal interaction is, as a rule, considerably smaller than the region $d_G$ of the damage that is capable of being generated by photodisruption, i.e. $d_T < d_G$, for which reason the region of thermal interaction $d_T$ is of no consequence or is not observable at all.

It therefore follows that fs radiation, the generation of which is complex and expensive, gives rise to no interactive processes that are fundamentally different from those of other short-time or ultra-short-time laser pulses, for instance those within the picosecond range. Only the dominance of the individual processes shifts: a qualitative jump at the transition from fs pulses to ps pulses, as often postulated in specialist circles, is, according to the findings of the inventors, non-existent.

For the generation of an incision in human ocular tissue the use of fs laser pulses which are difficult to generate is therefore not absolutely essential. Accordingly, within the scope of the invention use is made of ps pulses, which can be generated with considerably less effort and at lower costs. The invention is defined in the appended claims.

In embodiments of the invention a laser device is configured in such a way that its operating parameters are set or capable of being set to values with which equivalent or even still better accuracies can be obtained than was possible hitherto with fs laser pulses as regards the interaction of the laser radiation with the human ocular tissue being treated. For this purpose the invention exploits various findings which will be elucidated in more detail in the following.

1. The threshold (fluence threshold $F_{th}$) for the LIOB in the case of single pulses declines with declining pulse duration, to be specific, down to about 1 ps in proportion to the square root of the pulse duration, cf. US 5,656,186, but then noticeably more weakly within the range from 1 ps to about 10 fs. Though it has been recognised that at about 1 ps there is no abrupt point of inflection as claimed in this US patent, but rather that, instead of this, a monotonically falling decrease in the threshold $F_{th}$ obtains also for pulse durations below 1 ps. The dependence of the threshold $F_{th}$ on the square root of the pulse duration results from a deposition of thermal energy by the laser pulse and from a competing conduction of heat in the direction away from the focus location.

2. The LIOB threshold in the case of fs laser pulses appears to be defined more clearly and to be more deterministic than in the case of ns pulses; in the case of the latter, imperfections in the conduction band and defects in the material presumably play a significant role, since they supply the initial electrons which are necessary for avalanche ionisation and for the following generation of plasma and consequently for the process of photodisruption. In the case of fs laser pulses, on the other hand, the initial electrons are predominantly supplied by the multi-photon ionisation and are thereby coupled to the intensity of the laser radiation (i.e. for given focus diameter and given pulse duration, to the fluence). The genesis of the initial electrons therefore depends less on random defects and is more determinate. Therefore there is a sharper, more clearly determinable LIOB threshold.

3. The extent of the shock-wave generated by the photodisruption depends primarily on the laser-pulse energy. The bubble radius of the cavitation is proportional to the cube root of the laser-pulse energy. Only on account of the normally higher LIOB threshold for ps laser pulses or ns laser pulses, therefore, is the bubble radius and hence the damage zone greater in the case of the longer pulses.

4. Fs pulses with shorter wavelengths require lower pulse energies than such pulses with longer wavelengths in order to obtain equal LIOB damage and equal incision qualities in the ocular tissue. This is also corroborated by experiments that were carried out by the inventors with UV fs laser pulses at a wavelength of 345 nm. For incisions in the corneal tissue with a wavelength of 345 nm, only about 10 percent to 20 percent, for example, of the fs pulse energy is required compared to that at 1035 nm.

5. A repeated application of fs laser pulses or ps laser pulses onto the same location reduces the threshold for LIOB damage to the material at this location, i.e. it lowers the requisite fluence that a laser pulse must have in order to generate, after application of several such laser pulses, a lasting site of damage in the ocular tissue that is suitable for the generation of an incision. Every fs laser pulse or ps laser pulse lying below the fluence threshold for single-pulse damage leaves behind in the machined material a lattice imperfection or a thermal disturbance which accumulates in the course of application of several pulses and after a certain number of pulses results in the desired damage. The fluence per pulse required in this case lies clearly below the LIOB threshold for a single pulse.

6. Causes for a permanent material irritation upon beaming in laser pulses below the single-pulse damage threshold are primarily thermal changes. The higher the pulse repetition rate, the more strongly does the cumulative heating process arise upon multiple application of laser pulses onto the same location. The dissipation-time of the heat left behind that has been introduced by each laser pulse depends on the size of the focus volume (zone of interaction) and lies typically within the range of a few $\mu$s.

In the case of the ultimately occurring damage required for the separation of tissue it is then no longer a question - in the estimation of the inventors - of a pure optical photodisruption, as in the case of an optical single-pulse breakdown, but rather of a thermally assisted (key word: absorption) and defect-assisted optical damage by the fs laser pulses or ps laser pulses. The partial absorption of the pulse energy in the case of multiple-pulse application lowers the damage threshold, for example by a factor of ten, for instance in the case of application in the human cornea from about 1 $\mu$J to 100 nJ. These pulse energies lie not only within the availability range of cavity-dumped and long-cavity fs oscillators but also within the operating range of simple ps microchip lasers.

7. The accuracy of the machining or the extent of the damage zone depends not only on the laser-pulse energy, on the laser-pulse duration and on the number of laser pulses having an effect, but also on the focusing (i.e. on the focus volume, defined by the focus diameter or/and the focus length or the Rayleigh length) and consequently on the numerical aperture NA of the focusing optics being used. The threshold energy necessary for an LIOB depends greatly on the focus volume into which the energy of the laser pulse in question is introduced. At a given intensity threshold $I_{th}$ for the LIOB the pulse energy $E_{th}$ needed for this depends approximately as follows on the numerical aperture NA, on the pulse duration $\tau_L$ and on the wavelength $\lambda$:

$$E_{th} = \frac{I_{th}\tau_L\lambda^2}{\pi NA^2}$$

The volume into which the pulse energy is introduced depends furthermore on the absorption length $1/\alpha$ and on the thermal-diffusion length $L_T$ of the irradiated material, where $\alpha$ denotes a linear or non-linear absorption coefficient and $L_T$ depends on the thermal-diffusion coefficient of the material. High repetition-rates of the laser pulses beamed in and a low thermal-diffusion rate (such as, for example, in transparent dielectrics or in human ocular tissue) result in a strongly localised accumulation of the heat upon beaming in several consecutive pulses, the fluence of which lies in each instance below the threshold for a single-pulse LIOB. A strong absorption of the wavelength (e.g. $\alpha \geq 1000$ cm$^{-1}$) also causes the pulse energy to be substantially completely absorbed over, for example, a line segment of $1/\alpha \leq 10$ $\mu$m. This is, for example, the case for the excimer laser (193 nm), where despite a comparatively long pulse duration of, for example, 10 ns a sufficiently well localised, almost ather-mal, ablation of corneal tissue is achievable without the surrounding tissue being significantly impaired in the process.

In the case of laser parameters that were suitably chosen for a certain material or tissue, under certain circumstances the machining dimensions barely depend on the laser-pulse duration, so that instead of fs laser pulses use may also be made of longer laser pulses within the ps range. These are, as a rule, distinctly easier to generate. The inventors have discovered that, at any rate in the case of human ocular tissue, with ps laser pulses incision outcomes can be achieved that are comparable to those in the case of fs pulses.

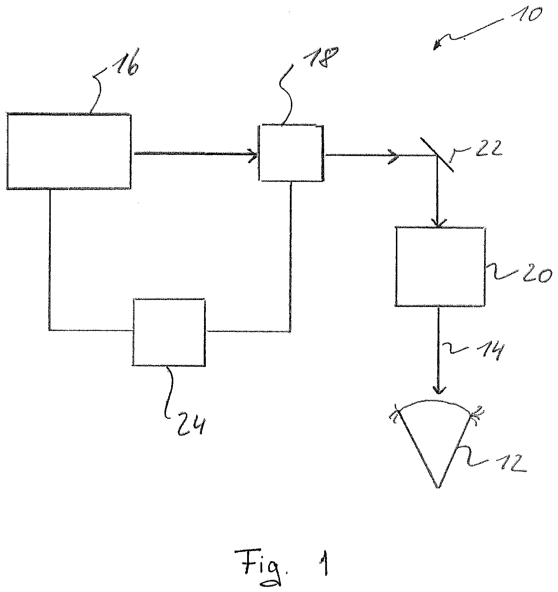

In the following an embodiment of a laser device will be described with regard to the appended Figure 1. The Figure shows schematically a laser device 10 that is intended for laser-surgical treatments of the cornea or lens of a human eye which is represented schematically at 12. Said laser device emits pulsed laser radiation (indicated at 14) with pulse durations within the picosecond range, the pulse duration of each emitted radiation pulse preferentially being longer than 10 ps and shorter than 300 ps, for example not longer than 100 ps. The laser radiation 14 is generated by a laser source 16 which, for example, includes a microchip laser, for instance of the VCSEL, VECSEL or MIXSEL type. It will be understood that other types of laser that are suitable for generating ps laser pulses may equally be used. The laser radiation emitted by the laser source 16 may be UV radiation within the range from 300 nm to 400 nm, for example within the range from 340 nm to 360 nm. Alternatively, the radiation generated by the laser source 16 may have a wavelength within the NIR range between 800 nm and 1100 nm, for example between 1000 nm and 1070 nm. In the case of an emitted UV wavelength it is conceivable that the latter is generated in the laser source 16 by frequency multiplication from a longer wavelength; for example, it is conceivable that the laser source 16 generates an emitted wavelength of 347 nm by generation of the third harmonic from a fundamental wavelength of 1040 nm.

The radiation pulses emitted by the laser source 16 have an energy between 1 nJ and 10 $\mu$J, whereby the pulse energy may vary, inter alia, in a manner depending on whether the separation of tissue to be realised in the tissue of the eye 12 by means of the laser radiation 14 is to be brought about by a single laser pulse per site of damage or by a plurality of laser pulses per site of damage. For single-pulse damage in the human corneal or lenticular tissue, a pulse energy within the range from 100 nJ to 10 $\mu$J may be preferred; for multiple-pulse damage, on the other hand, a pulse energy within the range from 10 nJ to 100 nJ.

Within the path of propagation of the radiation pulses emitted by the laser source 16 suitable means are located for beam shaping and beam control, which in the exemplary case shown are constituted by a scanner arrangement 18 indicated as a single functional block, a focusing objective 20 and also a deflecting mirror 22. The focusing objective 20 permits the radiation pulses to be focused onto a desired location on or in the eye 12, this location preferentially being situated on or in the cornea or in the lens of the eye 12. The scanner arrangement 18 serves to direct the focus location of the radiation pulses in the transverse radiation and, where appropriate, additionally in the longitudinal direction (relative to the direction of propagation of the laser radiation in the region of the eye 12) in such a way that the target region to be treated in the eye 12 is swept by means of the laser radiation 14. For the purpose of transverse focus control the scanner arrangement 18 may, for example, include, in a manner known as such, a pair of galvanometrically operable scanner mirrors that are capable of being swivelled about mutually perpendicular tilt axes. For the purpose of longitudinal control of the focus position the scanner arrangement 18 may, for example, include a lens element that is displaceable in the direction of propagation of the radiation or that is adjustable as regards its refractive power, by means of which the divergence of the radiation bundle beamed into the focusing objective 20 is capable of being changed.

A central control unit 24 controls the laser source 16 and the scanner arrangement 18 in accordance with a control program which is not represented in any detail. This control program represents an incision figure to be generated in the cornea or lens of the eye 12 by means of the laser radiation 14, this incision figure defining, for example, a corneal flap to be generated for a LASIK treatment. It will be understood that, depending on the requisite form of treatment, the incision figure may have a varying geometry and position in the cornea or in the lens.

The laser device 10 has been configured with regard to its operating parameters in such a way that the radiation pulses impinging onto the eye 12 at the focus location can give rise to a laser-induced optical breakdown in the tissue of the eye 12, which under certain circumstances may be accompanied by initial thermal partial damage to the piece of tissue in question. Such a thermal component of the ultimate tissue damage may, in particular, be observable when the wavelength of the laser radiation 14 lies within the range between 1000 nm and 1100 nm or/and the laser pulses impinge onto the eye 12 in the form of pulse groups and in each instance have a fluence that lies below the fluence threshold for single-pulse damage. In this connection the term 'fluence' designates the pulse energy per surface area in the region of the beam waist (focus) of the laser radiation 14. For the case of intended single-pulse damage, i.e. a single laser pulse per site of damage, the fluence preferentially lies above 1 J/cm$^2$ right up to 10 J/cm$^2$, and for intended multiple-pulse damage, i.e. a plurality of pulses per site of damage, it lies below 1 J/cm$^2$ right down to, for example, about 0.1 J/cm$^2$. By stringing together such single-pulse sites of damage or/and multiple-pulse sites of damage, incisions having diverse shapes can be generated in the cornea or in the lens.

The focus diameter of the emitted laser radiation 14 lies, for example, within the range from 1 $\mu$m to 10 $\mu$m and is preferably less than 5 $\mu$m. In addition to the pulse length, the wavelength, the pulse energy, the pulse repetition rate, the fluence, the focus diameter and the number of pulses per site of damage, the scanning speed at which the scanner arrangement 18 traverses the focus position transversely or/and longitudinally in the course of machining the eye 12 may also be a suitable configuration parameter in order to configure the laser device 10 in such a way that the single pulses or pulse groups emitted from it in each instance give rise to the tissue damage in the eye 12 that is desired for the generation of an incision. Suitable values for these parameters will be ascertained, for example, within the scope of experiments on pigs' eyes. On the basis of the findings acquired within the scope of these experiments, the manufacturer configures the laser device 10 in such a way that the laser device 10 enables operation with a suitable set of parameter values with which the desired tissue damage is ensured. The starting-point in the ascertainment of such a set of parameter values is always a pulse duration that is selected within the picosecond range, preferably from the two-digit or three-digit picosecond range. The inventors have discerned that in the case of pulse durations within the ps range various coupled physical effects ensure that the fluence threshold for the desired damage effect can be lower than hitherto presumed, at any rate for pure LIOB damage with ps pulses (especially single pulses). In particular, the inventors have discerned that in the case of ps laser pulses a damage effect in the ocular tissue that is suitable for the generation of an incision (with minimal side-effects) can be obtained already with values of the fluence that otherwise were considered possible only for ultra-short fs laser pulses (for example, within the range from 100 fs to 400 fs).

According to one configuration, an exploitation of coupled physical effects may, for example, consist in use being made, for the ps laser pulses, of a UV wavelength (e.g. between 300 nm and 400 nm) and, at the same time, in a multiple-pulse application being undertaken in which a pulse train consisting of several pulses is beamed onto the same tissue location in order, with the accumulated individual action of each pulse, to leave behind overall a site of damage in the ocular issue. The UV wavelength is associated with a partial (linear) initial absorption in the ocular issue (e.g. from 1 percent to 30 percent) and consequently facilitates the start of the damage process. Expressed differently, with otherwise identical operating parameters of the laser device 10 the fluence threshold for the desired damage is lower if the wavelength lies within the UV region instead of within the NIR region. In the case of multiple-pulse application, the fluence of each laser pulse lies below the threshold for single-pulse damage in the ocular issue; in this connection, each pulse leaves behind a thermally induced defect in the ocular tissue which lowers the damage threshold (i.e. the threshold from which a damage effect required for the generation of an incision appears in the ocular tissue) in comparison with single-pulse damage.

By combination of a UV wavelength and a multiple-pulse application in this way, it is possible, for example, for a fluence threshold for the desired tissue damage to be obtained that is distinctly lower than in the case of a single-pulse application within the NIR wavelength region. A few examples of requisite fluence values are specified below, which were ascertained by the inventors within the scope of a rough estimation for various combinations of the parameters constituted by pulse duration, wavelength and number of pulses N per site of damage, in which connection these fluence values represent thresholds from which a damage effect required for the generation of an incision in the ocular tissue appears with the necessary statistical confidence:

N = 1, 1030 nm, 100 fs: about 2 J/cm$^2$
N = 1, 345 nm, 100 fs: about 1.0 J/cm$^2$
N = 10$^3$, 1030 nm, 100 fs: about 0.5 J/cm$^2$
N = 10$^3$, 345 nm, 100 fs: about 0.1 J/cm$^2$
N = 1, 1030 nm, 100 ps: about 10 J/cm$^2$
N = 1, 345 nm, 100 ps: about 5 J/cm$^2$
N = 10$^3$, 1030 nm, 100 ps: about 2 J/cm$^2$
N = 10$^3$, 345 nm, 100 ps: about 0.5 J/cm$^2$

The above numerical values for the approximate fluence thresholds in optically transparent materials (such as human corneal or lenticular tissue) show clearly that the damage thresholds for fs laser pulses and ps laser pulses, given appropriate choice of the wavelength and of the number of pulses N, may be the same, even though the pulse durations are different by a factor of 10$^3$. The damage effect in the human ocular tissue that is needed for the generation of an incision can consequently be realised with ps laser pulses at roughly identical pulse energies (with given focusing, i.e. given focus diameter) as with fs pulses. Since the thermomechanical collateral damage to the surrounding tissue by virtue of pressure wave and conduction of heat is principally determined by the pulse energy that is used, with ps laser pulses it is possible to obtain slight side-effects in the surrounding tissue outside the immediate focus region, which are comparable to those obtained with fs laser pulses. By choice of suitable values for the wavelength, the pulse duration and the number of pulses per site of damage, with ps lasers similarly low fluence thresholds (and correspondingly low energy thresholds) can consequently be obtained as can be obtained with the fs single pulses commonly regarded as only slightly damaging. The number of pulses per site of damage may amount in this case to, for example, up to $10^4$. The invention accordingly enables the use of comparatively compact and inexpensive ps laser systems for treatments (laser-assisted ophthalmological treatments) for which hitherto predominantly only fs laser systems were taken into consideration.

A laser device in accordance with a preferred embodiment outputs focused picosecond laser radiation and may be adjustable for at least one the wavelength of the laser radiation and the number N of radiation pulses (i.e. number of pulses per burst) applied to substantially the same location of the tissue being treated for generating one site of damage in the tissue. In embodiments of the present invention, the pulse energy of the radiation pulses may be anywhere below 5 μJ, advantageously below 1 μJ. The pulse length of each radiation pulse may be anywhere between 1 ps and 500 ps in embodiments of the present invention. It has been found that by using a laser device in accordance with the present disclosure, incisions can be generated in human eye tissue by stringing together a plurality of local sites of damage, each site of damage including a laser induced optical breakdown of the eye tissue being irradiated with the laser radiation, wherein surrounding damage by thermal or micromechanical effects due to e.g. pressure waves (which may penetrate deep into the eye and even reach the retina) can be substantially avoided. Specifically, it has been found that by using a laser device in accordance with the present disclosure a similarly high cutting precision and a similarly small-sized damage zone can be achieved as with a conventional laser device operating with fs laser radiation pulses.

## Claims

1. Process for configuring a laser device (10) adapted to emit focused pulsed laser radiation (14) and intended for use in laser-assisted treatments of the human eye (12), the process comprising the following steps:

   - selecting a pulse length within a range from above 10 ps to below 300 ps,
   - selecting a wavelength for the laser radiation within one of a range from 300 nm to 400 nm and a range from 800 nm to 1100 nm,
   - performing experiments on PMMA test plates to ascertain such a set of parameter values of the laser device that when the laser device is operated with these parameter values a two-dimensionally extensive separation of tissue of human corneal or lenticular tissue is achievable by means of the laser radiation by stringing together a plurality of local sites of damage, each local site of damage including a laser induced optical breakdown of the tissue, the set of parameter values including, in addition to the selected pulse length and the selected wavelength, values for at least one of the following parameters: a pulse energy, a pulse repetition rate, a fluence per radiation pulse, a number of pulses per site of damage, a scanning speed of a scanning apparatus (18) of the laser device, a focus diameter,
   - configuring the laser device in such a manner that operation of the laser device with the ascertained set of parameter values is enabled,
   wherein the set of parameter values is ascertained for a plurality of radiation pulses per site of damage and the fluence per radiation pulse is selected below a fluence threshold for single-pulse damage.

2. Process according to Claim 1, wherein for the ascertainment of the set of parameter values the fluence per radiation pulse is selected within the range from 0.1 J/cm$^2$ to 1 J/cm$^2$.

3. Process according to one of the preceding claims, wherein for the ascertainment of the set of parameter values the pulse energy is selected within the range from 1 nJ to 10 μJ, preferentially within the range from 100 nJ to 1 μJ.

4. Process according to one of the preceding claims, wherein for the ascertainment of the set of parameter values the pulse repetition rate is selected within the range from 10 kHz to 1 GHz, preferentially within the range from 100 kHz to 100 MHz.

5. Process according to one of the preceding claims, wherein the laser device for generating the laser radiation (14) is equipped with a mode-coupled or Q-switched semiconductor laser or solid-state laser or with a microchip laser or with a vertical-cavity surface-emitting laser or with a vertical-external-cavity surface-emitting laser or with a mode-locked integrated-external-cavity surface-emitting laser.

6. A laser device (10) for human eye surgery, comprising:

   - a source (16) for providing a beam of laser radiation;
   - a scanner (18) for scanning the beam of laser radiation;

- focusing optics (20) for focusing the beam of laser radiation;
- a control program representing an incision figure to be generated in a human eye; and
- a control device (24) coupled to the scanner for controlling the scanning of the beam of laser radiation in accordance with the control program;

wherein the source includes one of the following for generating the beam of laser radiation: a mode-coupled semiconductor laser, a mode-coupled solid-state laser, a Q-switched semiconductor laser, a Q-switched solid-state laser, a microchip laser, a vertical-cavity surface-emitting laser, a vertical-external-cavity surface-emitting laser, a mode-locked integrated-external-cavity surface-emitting laser;

wherein the laser radiation provided by the source has radiation parameter values permitting a two-dimensionally extensive separation of tissue of human corneal or lenticular tissue by means of the laser radiation by stringing together a plurality of local sites of damage, each local site of damage including a laser induced optical breakdown of the tissue, the laser radiation having a pulse length within a range from above 10 ps to below 300 ps, and a wavelength within one of a range from 300 nm to 400 nm and a range from 800 nm to 1100 nm, and wherein the radiation parameter values are set for a plurality of radiation pulses per site of damage and the fluence per radiation pulse is set below a fluence threshold for single-pulse damage.

**Patentansprüche**

1. Verfahren zum Konfigurieren einer Laservorrichtung (10), die zum Emittieren von fokussierter gepulster Laserstrahlung (14) eingerichtet und zur Verwendung in laserunterstützten Behandlungen des menschlichen Auges (12) vorgesehen ist, wobei das Verfahren die folgenden Schritte umfasst:

- Auswählen einer Pulslänge innerhalb eines Bereichs von über 10 ps bis unter 300 ps,
- Auswählen einer Wellenlänge für die Laserstrahlung innerhalb einem von einem Bereich von 300 nm bis 400 nm und einem Bereich von 800 nm bis 1100 nm,
- Durchführen von Experimenten an PMMA-Testplatten, um einen solchen Satz von Parameterwerten der Laservorrichtung festzustellen, dass, wenn die Laservorrichtung mit diesen Parameterwerten betrieben wird, eine zweidimensionale extensive Trennung von Gewebe des menschlichen Cornea- oder Linsengewebes mittels der Laserstrahlung erreichbar ist, indem eine Vielzahl lokaler Schadensstellen aneinandergereiht wird, wobei jede lokale Schadensstelle einen laserinduzierten optischen Abbau des Gewebes einschließt, wobei der Satz von Parameterwerten zusätzlich zu der ausgewählten Pulslänge und der ausgewählten Wellenlänge Werte für mindestens einen der folgenden Parameter einschließt: eine Pulsenergie, eine Pulswiederholungsrate, eine Fluenz pro Strahlungspuls, eine Anzahl von Pulsen pro Schadensstelle, eine Scangeschwindigkeit eines Scanapparats (18) der Laservorrichtung, einen Fokusdurchmesser,
- Konfigurieren der Laservorrichtung in einer solchen Weise, dass Betrieb der Laservorrichtung mit dem festgestellten Satz von Parameterwerten ermöglicht wird,
wobei der Satz der Parameterwerte für eine Vielzahl von Strahlungspulsen pro Schadensstelle festgestellt wird und die Fluenz pro Strahlungspuls unterhalb eines Fluenzschwellenwerts für Einzelpulsschäden ausgewählt wird.

2. Verfahren nach Anspruch 1, wobei zur Feststellung des Satzes von Parameterwerten die Fluenz pro Strahlungspuls innerhalb des Bereichs von 0,1 J/cm$^2$ bis 1 J/cm$^2$ ausgewählt wird.

3. Verfahren nach einem der vorhergehenden Ansprüche, wobei zur Feststellung des Satzes von Parameterwerten die Pulsenergie innerhalb des Bereichs von 1 nJ bis 10 μJ, vorzugsweise innerhalb des Bereichs von 100 nJ bis 1 μJ ausgewählt wird.

4. Verfahren nach einem der vorhergehenden Ansprüche, wobei zur Feststellung des Satzes von Parameterwerten die Pulswiederholungsrate innerhalb des Bereichs von 10 kHz bis 1 GHz, vorzugsweise innerhalb des Bereichs von 100 kHz bis 100 MHz ausgewählt wird.

5. Verfahren nach einem der vorhergehenden Ansprüche, wobei die Laservorrichtung zum Generieren der Laserstrahlung (14) mit einem modusgekoppelten oder Q-geschalteten Halbleiterlaser oder Festkörperlaser oder mit einem Mikrochiplaser oder mit einem oberflächenemittierenden, vertikalen Hohlraum aufweisenden (VCSE)-Laser oder mit einem oberflächenemittierenden, vertikalen äußeren Hohlraum aufweisenden (VECSE)-Laser oder mit einem modusverkoppelten oberflächenemittierenden, integrierten äußeren Hohlraum aufweisenden Laser ausgestattet ist.

6. Laservorrichtung (10) für Chirurgie am menschlichen Auge, umfassend:

- eine Quelle (16) zum Bereitstellen eines Strahls der Laserstrahlung;
- einen Scanner (18) zum Scannen eines Strahls der Laserstrahlung;
- Fokussieroptik (20) zum Fokussieren eines Strahls der Laserstrahlung;
- ein Steuerprogramm, das eine in einem menschlichen Auge zu generierende Inzisionsfigur repräsentiert; und
- eine Steuervorrichtung (24), die an den Scanner gekoppelt ist, um das Scannen des Strahls der Laserstrahlung gemäß dem Steuerprogramm zu steuern;
wobei die Quelle eines der folgenden zum Generieren eines Strahls der Laserstrahlung einschließt: einen modusgekoppelten Halbleiterlaser, einen modusgekoppelten Festkörperlaser, Q-geschalteten Halbleiterlaser, einen Q-geschalteten Festkörperlaser, einen Mikrochiplaser, einen oberflächenemittierenden, vertikalen Hohlraum aufweisenden (VCSE)-Laser oder einen oberflächenemittierenden, vertikalen äußeren Hohlraum aufweisenden (VECSE)-Laser oder einen modusverkoppelten oberflächenemittierenden, integrierten äußeren Hohlraum aufweisenden Laser;
wobei die durch die Quelle bereitgestellte Laserstrahlung Strahlungsparameterwerte aufweist, die eine zweidimensionale extensive Trennung des Gewebes des menschlichen Cornea- oder Linsengewebes mittels der Laserstrahlung ermöglicht, indem eine Vielzahl lokaler Schadensstellen aneinandergereiht wird, wobei jede lokale Schadensstelle einen laserinduzierten optischen Abbau des Gewebes einschließt, wobei die Laserstrahlung eine Pulslänge innerhalb eines Bereichs von über 10 ps bis unter 300 ps und eine Wellenlänge innerhalb einem von einem Bereich von 300 nm bis 400 nm und einem Bereich von 800 nm bis 1100 nm einschließt, und wobei die Strahlungsparameterwerte für eine Vielzahl von Strahlungspulsen pro Schadensstelle festgelegt werden und die Fluenz pro Strahlungspuls unter einen Fluenzschwellenwert für Einzelpulsschaden festgelegt wird.

## Revendications

1. Procédé de configuration d'un dispositif laser (10) adapté pour émettre un rayonnement laser pulsé focalisé (14) et destiné à être utilisé dans des traitements assistés par laser de l'oeil humain (12), le procédé comprenant les étapes suivantes :

- sélectionner une longueur d'impulsion dans une gamme de plus de 10 ps à moins de 300 ps,
- sélectionner une longueur d'onde pour le rayonnement laser dans une gamme de 300 nm à 400 nm ou une gamme de 800 nm à 1100 nm,
- effectuer des expériences sur des plaques d'essai en PMMA pour établir un ensemble de valeurs de paramètres du dispositif laser tel que, quand le dispositif laser fonctionne avec ces valeurs de paramètres, une séparation bidimensionnellement extensive de tissu cornéen ou lenticulaire humain puisse être obtenue au moyen du rayonnement laser en enchaînant une pluralité de sites locaux de dommage, chaque site local de dommage comportant une fragmentation optique induite par laser du tissu, l'ensemble de valeurs de paramètres comportant, en plus de la longueur d'impulsion sélectionnée et de la longueur d'onde sélectionnée, des valeurs pour au moins un des paramètres suivants : une énergie d'impulsion, une fréquence de répétition d'impulsion, une fluence par impulsion de rayonnement, un nombre d'impulsions par site de dommage, une vitesse de balayage d'un appareil de balayage (18) du dispositif laser, un diamètre de focalisation,
- configurer le dispositif laser de manière à ce que le fonctionnement du dispositif laser avec l'ensemble établi de valeurs de paramètres soit autorisé,
dans lequel l'ensemble de valeurs de paramètres est établi pour une pluralité d'impulsions de rayonnement par site de dommage et la fluence par impulsion de rayonnement est sélectionnée au-dessous d'un seuil de fluence pour un dommage par une seule impulsion.

2. Procédé selon la revendication 1 dans lequel, pour l'établissement de l'ensemble de valeurs de paramètres, la fluence par impulsion de rayonnement est sélectionnée dans la gamme de 0,1 J/cm$^2$ à 1 J/cm$^2$.

3. Procédé selon une des revendications précédentes dans lequel, pour l'établissement de l'ensemble de valeurs de paramètres, l'énergie d'impulsion est sélectionnée dans la gamme de 1 nJ à 10 µJ, de préférence dans la gamme de 100 nJ à 1 µJ.

4. Procédé selon une des revendications précédentes dans lequel, pour l'établissement de l'ensemble de valeurs de paramètres, la fréquence de répétition d'impulsion est sélectionnée dans la gamme de 10 kHz à 1 GHz, de préférence dans la gamme de 100 kHz à 100 MHz.

5. Procédé selon une des revendications précédentes, dans lequel le dispositif laser destiné à générer le rayonnement laser (14) est équipé d'un laser à semi-conducteur ou laser solide à modes couplés ou à commutation Q ou d'un laser à micropuce ou d'un laser à cavité verticale émettant par la surface ou d'un laser à cavité verticale externe émettant par la surface ou d'un laser à cavité externe intégrée émettant par la surface à modes verrouillés.

6. Dispositif laser (10) pour la chirurgie de l'oeil humain, comprenant :

- une source (16) pour délivrer un faisceau de rayonnement laser ;
- un appareil de balayage (18) pour balayer le faisceau de rayonnement laser ;
- une optique de focalisation (20) pour focaliser le faisceau de rayonnement laser ;
- un programme de contrôle représentant une figure d'incision devant être générée dans un oeil humain ; et
- un dispositif de contrôle (24) couplé à l'appareil de balayage pour contrôler le balayage du faisceau de rayonnement laser en fonction du programme de contrôle ;
dans lequel la source comporte un des éléments suivants pour générer le faisceau de rayonnement laser : un laser à semi-conducteur à modes couplés, un laser solide à modes couplés, un laser à semi-conducteur à commutation Q, un laser solide à commutation Q, un laser à micropuce, un laser à cavité verticale émettant par la surface, un laser à cavité verticale externe émettant par la surface, un laser à cavité externe intégrée émettant par la surface à modes verrouillés ;
dans lequel le rayonnement laser délivré par la source a des valeurs de paramètres de rayonnement permettant une séparation bidimensionnellement extensive de tissu cornéen ou lenticulaire humain au moyen du rayonnement laser par enchaînement d'une pluralité de sites locaux de dommage, chaque site local de dommage comportant une fragmentation optique induite par laser du tissu, le rayonnement laser ayant une longueur d'impulsion dans une gamme de plus de 10 ps à moins de 300 ps, et une longueur d'onde dans une gamme de 300 nm à 400 nm ou une gamme de 800 nm à 1100 nm, et les valeurs de paramètres de rayonnement étant réglées pour une pluralité d'impulsions de rayonnement par site de dommage et la fluence par impulsion de rayonnement étant réglée au-dessous d'un seuil de fluence pour un dommage par une seule impulsion.

Fig. 1

**EP 2 836 175 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1787607 A1
- US 20040243111 A
- US 5520679 A
- EP 2345394 A
- WO 2008151616 A1
- US 5656186 A